# EUROPEAN PATENT SPECIFICATION

(11) **EP 0 269 390 B1**
(45) Date of publication and mention of the grant of the patent: **25.08.1993**
(21) Application number: 87310285.9
(22) Date of filing: 20.11.1987
(51) Int. Cl.: C12P 21/02

(54) **Enzymatic L-aspartyl-L-phenylalanine alkyl ester production**
Enzymatische Herstellung von L-Aspartyl-L-Phenylalanin-Alkylester
Production enzymatique de l'ester alcoylique de L-aspartyl-L-phénylalanine

(30) Priority: 21.11.1986 US 933711
(43) Date of publication of application: 01.06.1988
(73) Proprietor: GENENCOR INTERNATIONAL, INC., South San Francisco, CA 94080 (US)
(72) Inventor: Boston, Matthew G., Richmond, CA 94801 (US); Poulose, Ayrookaran J., San Bruno, CA 94066 (US)
(74) Representative: Armitage, Ian Michael

(56) References cited:
- EP-A- 0 149 594
- WO-A-88/01650
- FR-A- 2 453 137
- US-A- 4 086 136
- CHEMICAL ABSTRACTS, vol. 103, no. 23, 9th December 1985, page 579, no. 194943y, Columbus, Ohio, US; && JP-A-60 41 499 (ASAHI CHEMICAL INDUSTRY CO., LTD) 05-03-1985

## Description

This invention relates to a method of producing L-aspartyl-L-phenylalanine alkyl ester (abbreviated as APM hereafter).

APM is a peptide which is noted as a sweetener in recent years. It is well known that the processes for production of APM or L-aspartyl-L-phenylalanine (AP) include a chemical synthesizing process and an enzymatic synthesizing process.

The chemical synthesizing process for the production of APM comprises condensing N-protected L-aspartic acid anhydride and L-phenylalanine methylester (PM) to obtain N-protected APM. The protective group is removed afterwards. The enzymatic synthesizing process comprises exerting the effect of a protein-decomposing enzyme on N-protected L-aspartic acid and PM to obtain N- protected APM or the PM adduct of N-protected APM and then removing the protective group to form APM. However, both processes require the complicated steps of introducing the protective groups and removing the same. (See GB2,092,161A).

The art also teaches a process for producing APM without using protective groups (JP 83/126,796) which is a microbiological synthetic process using one of Pseudomonas, Torulopsis, Rhodotoryla, and Sporobolomyces, but this is not always suitable for industrial production because of extremely low yields.

A more direct unprotected route is employed wherein a microorganism or an enzyme therefrom can bring around a direct formation of APM from L-aspartic acid and PM. (Japanese Patent application No. 75559/1983). However, a difficulty of this process for producing APM using L-aspartic acid without a protective group is that the reaction of L-aspartic acid and PM to form APM is an equilibrium reaction, and the equilibrium prevents the substrates from being converted efficiently to APM thus giving poor yields. While some improvement in yield was achieved in later references using the L-phenylalanine ester residue of an alcohol (EP 154,472) the reaction remains equilibrium driven and hence relatively low yields occur.

The present invention improves the yields of production without the equilibrium problems of the unblocked route or routes employing nonderivatized aspartic acid.

Accordingly, the invention relates to a method of producing L-aspartyl-L-phenylalanine alkyl esters comprising reacting in a an organic solvent-containing, optionally aqueous medium, non-N-protected L-aspartic acid alpha ester (LAE) or L-aspartic acid alpha amide (LAA) with L-phenylalanine alkyl ester (LPAE) in the presence of an enzyme, microorganism containing the enzyme or enzyme containing fraction of the microorganism or enzyme immobilized to solid supports where the enzyme is capable of forming non-N-protected L-aspartyl-L-phenylalanine alkyl ester by condensation of L-aspartic acid alpha ester or L-aspartic acid alpha amide and L-phenylalanine alkyl ester.

The invention relates to the production of APM starting from L-aspartic acid alpha ester or alpha and and L-phenylalanine alkyl ester. Alpha esters of L-aspartic acid are well known in the art and can be made for example by the methods of J. Kovacs et.al., J. Org. Chem. 26, 1084 (1961). Preferred alpha esters include a residue of an alcohol, a substituted or unsubstituted phenol, a thiol or an alkyl ester; also, it is contemplated that other forms of L-aspartic acid alpha ester or alpha amide could be used. A preferred other form would be L-aspartic acid dialkylester. Such compounds are known within the art. Such additional groups could then be removed by means known in the art. Alpha amides are made by reacting the aspartic alpha methyl ester with ammonia in methanol under conditions known in the art. Other methods are known in the art for making amides and can also be utilized. Amide group at the alpha position can be derived from, for example, NH₃ or R-NH₂ or R₂NH or R₃N where R contains an aromatic or aliphatic structure. Preferred amide is aspartic amide. Preferred alkyl ester is the methyl ester. L-pehnylalanine alkyl esters are well known in the art especially the lower (1-6) alkyl esters. A preferred lower alkyl ester of L-phenylalanine is the methyl ester.

The process is carried out by reacting L-aspartic acid alpha ester or alpha amide with L-phenylalanine alkyl ester in an aqueous organic media in the presence of an enzyme capable of condensing the alpha ester or alpha amide of L-aspartic acid to form the condensation product APM. Reactions are carried out at suitable temperatures, preferably within the range of 25-45° C for a period of about 1 to 10 days. The reaction is carried out at a pH of roughly 4-9. A preferred pH is about 8. The actual amounts of starting reactants are of course relative with approximately stoichiometric amounts reacting to form APM and preferably for optimum results such amounts at any relative concentration is preferred. However, more or less of one or the other reactant can be used as well as selecting more than one L-aspartic acid ester or amide or more than one L-phenylalanine alkyl ester. Enzymes selected preferably have a preference for the alpha ester or amide bonds of L-aspartic acid and will preferably not hydrolyze the bonds (especially ester) of L-phenylalanine alkyl ester and other L-aspartic acid bonds or the APM product.
A preferred enzyme is the extracellular protease (obtained from Sigma Chemical Co.) of Staphylococcus aureus strain V8 and mutant variants and genetically altered forms of that protease which retains the same function. The amino acid sequence of the N-terminal region of the enzyme was previously known (G.R. Drapeau, (1978) Canadian Journal of Biochemistry 56 534-44). However, the complete amino acid and encoding DNA sequence of the mature enzyme is given below (see also Carmona et al, Nucleic Acids Research, 15, 16, (1987), 6757).

GTT ATA TTA CCA AAT AAC GAT CGT CAC CAA ATC ACA GAT ACA ACG AAT GGT
1 Val Ile Leu Pro Asn Asn Asp Arg His Gln Ile Thr Asp Thr Thr Asn Gly

CAT TAT GCA CCC GTA ACT TAT ATT CAA GTT CAA GCA CCT ACT GGT ACA TTT
18 His Tyr Ala Pro Val Thr Tyr Ile Gln Val Glu Ala Pro Thr Gly Thr Phe

ATT GCT TCC GGT GTA GTT GTA GGT AAA GAT ACT CTT TTA ACA AAT AAA CAC
35 Ile Ala Ser Gly Val Val Val Gly Lys Asp Thr Leu Leu Thr Asn Lys His

GTC GTA GAT GCT ACG CAC GGT GAT CCT CAT GCT TTA AAA GCA TTC CCT TCT
52 Val Val Asp Ala Thr His Gly Asp Pro His Ala Leu Lys Ala Phe Pro Ser

GCA ATT AAC CAA GAC AAT TAT CCA AAT GGT GGT TTC ACT GCT GAA CAA ATC
69 Ala Ile Asn Gln Asp Asn Tyr Pro Asn Gly Gly Phe Thr Ala Glu Gln Ile

ACT AAA TAT TCA GGC GAA GGT GAT TTA GCA ATA GTT AAA TTC TCC CCT AAT
86 Thr Lys Tyr Ser Gly Glu Gly Asp Leu Ala Ile Val Lys Phe Ser Pro Asn

GAG CAA AAC AAA CAT ATT GGT GAA GTA GTT AAA CCA GCA ACA ATG AGT AAT
103 Glu Gln Asn Lys His Ile Gly Glu Val Val Lys Pro Ala Thr Met Ser Asn

AAT GCT GAA ACA CAA GTT AAC CAA AAT ATT ACT GTA ACA GGA TAT CCT GGT
120 Asn Ala Glu Thr Gln Val Asn Gln Asn Ile Thr Val Thr Gly Tyr Pro Gly

GAT AAA CCT GTA GCA ACA ATG TGG GAA AGT AAA GGA AAA ATC ACT TAC CTC
137 Asp Lys Pro Val Ala Thr Met Trp Glu Ser Lys Gly Lys Ile Thr Tyr Leu

AAA GGC GAA GCT ATG CAA TAT GAT TTA AGT ACA ACT GGT GGT AAT TCA GGT
154 Lys Gly Glu Ala Met Gln Tyr Asp Leu Ser Thr Thr Gly Gly Asn Ser Gly

TCA CCT GTA TTT AAT GAA AAA AAT GAA GTG ATC GGA ATT CAT TGG GGC GGT
171 Ser Pro Val Phe Asn Glu Lys Asn Glu Val Ile Gly Ile His Trp Gly Gly

GTA CCA AAT GAA TTT AAT GGT GCG GTA TTT ATT AAT GAA AAT GTA CGC AAC
188 Val Pro Asn Glu Phe Asn Gly Ala Val Phe Ile Asn Glu Asn Val Arg Asp

TTC TTA AAA CAA AAT ATT GAA GAT ATC CAT TTT GCC AAC GAT GAC CAA CCT
205 Phe Leu Lys Gln Asn Ile Glu Asp Ile His Phe Ala Asn Asp Asp Gln Pro

AAT AAC CCA GAT AAT CCT GAT AAC CCT AAC AAT CCT GAT AAC CCT AAC AAC
222 Asn Asn Pro Asp Asn Pro Asp Asn Pro Asn Asn Pro Asp Asn Pro Asn Asn

CCA GAT GAA CCA AAT AAC CCT GAC AAC CCT AAC AAC CCT GAT AAT CCA GAC
239 Pro Asp Glu Pro Asn Asn Pro Asp Asn Pro Asn Asn Pro Asp Asn Pro Asp

AAT GGC GAT AAC AAT AAT TCA GAC AAT CCA GAT GCA GCT
254 Asn Gly Asp Asn Asn Asn Ser Asp Asn Pro Asp Ala Ala

It is also known in the art that site specific changes in codons; i.e., other than naturally occurring or random mutations or alterations can be made to enzymes which can alter specific activity, reaction rate, reaction specificity Kcat, Km stability to organic solvents and temperature and the like. Such modifications for site specific changes at specific codons are well known in the art and intended to be a part of this invention when selecting enzymes. Important site specific changes would occur at or near the active site, for example near the catalytic residues of an enzyme, or the face of the enzyme, or at some other amino acid at or near the outer portion of the amino acid. Additionally, disulfide bonds can also be introduced for stabilizing the enzyme. Alternatively, the microorganism containing the enzyme or a fraction of the microorganism containing the enzyme may be used. Immobilized enzymes can also be used.

The reaction of the enzyme of LAE or LAA with PAE in the presence of the appropriate selected enzyme yields the condensation product APM.

Where enzymes are used from microorganisms or fractions of microorganisms, such microorganisms can be obtained by using ordinary culture media. Further, reactants may be added at the beginning or during the process of cultivation of the cells.

The culture media to be used for the microorganisms are ordinary ones containing usual carbon and nitrogen sources and inorganic ions. Moreover, the addition of trace amounts of organic nutritive substances such as vitamins and amino acid often brings about desirable results.

The carbon sources suitable for use herein include carbohydrates such as glucose and sucrose, organic acids such as acetic acid, and alcohols. The nitrogen sources suitable for use herein include ammonia gas, aqueous ammonia and ammonium salts. The inorganic ions are suitably selected from e.g. magnesium ion, phosphoric acid ion, potassium ions and iron ion, as necessary.

The cultures are suitably conducted under aerobic conditions at pH 4-9, preferably about pH 8, at suitable temperatures controlled within the range of 25-45° C, and for about 1-10 days.

The microorganisms which can be used in this invention include the whole culture solutions obtained after completion of cultivation, microorganisms separated from the culture solutions, or washed microorganisms. Also, the microorganisms which can be used may be freeze-dried, acetone-dried, contacted with toluene, surfactants, etc., treated with lysozyme, exposed to ultrasonic waves or mechanically ground, and enzyme protein fractions obtained from these cell-treating materials having enzymic activity. Fixed cells of these microorganisms, insolubilized materials of treated cells, etc. may also be used.

As aqueous media, there can be used those containing water, buffers, and organic solvents such as ethanol. Moreover, nutritive elements needed for the growth of microorganisms, anti-oxidants, surfactants, coenzymes, hydroxylamine and metallic ions, organic solvents such as DMSO etc. can be added to the aqueous media if necessary.

When the whole culture solutions, culture cells or treated cell materials of the above-mentioned microorganisms are brought directly into contact with the reactants to exert the action thereon, an aqueous medium is prepared by dissolving or suspending reactants and the culture solution, culture cells, or treated cell materials, and is controlled suitably at a temperature of 10-70° C and pH 4-9, and allowed to stand for awhile or stirred.

The APM thus produced can be separated and purified by the known separation processes. The APM obtained is determined with an amino-acid analyzer.

### Example 1

Aqueous solution of L-phenylalanine methyl ester and L-aspartic acid alpha methyl ester were made and adjusted to pH 8.0. These solutions were then mixed with Dimethyl Sulfoxide (DMSO) so that final concentration of L-phenylalanine methyl ester, L-aspartic acid alpha methyl ester and DMSO were 0.1M, 0.5M and 50% respectively. A solution of the V-8 enzyme described earlier was then added to a final concentration of 0.5 mg/ml and the reaction mixture allowed to stand at room temperature for 24 hours. Analysis by HPLC of the product based on PM converted to APM was about 33%.

## Claims

1. A method for producing L-aspartyl-L-phenylalanine alkyl esters comprising:
a) reacting in an organic solvent-containing medium non-N-protected L-aspartic acid alpha ester or L-aspartic acid alpha amide with L-phenylalanine alkyl ester in the presence of an enzyme, microorganism containing the enzyme, enzyme containing fraction of a microorganism, or enzyme immobilized on solid support, said enzyme being capable of forming L-aspartyl-L-phenylalanine alkyl ester by condensation of the non-N-protected L-aspartic acid alpha ester or L-aspartic acid alpha amide and L-phenylalanine alkyl ester.

2. A method according to claim 1 wherein the selected enzyme is the extracellular protease of Staphylococcus aureus strain V8, or a mutant variant or genetically altered form thereof which retains the same function.

3. A method according to claim 1 wherein the enzyme selected has an N-terminus corresponding to that of the amino acid sequence:

GTT ATA TTA CCA AAT AAC GAT CGT CAC CAA ATC ACA GAT ACA ACG AAT GGT
1 Val Ile Leu Pro Asn Asn Asp Arg His Gln Ile Thr Asp Thr Thr Asn Gly

CAT TAT GCA CCC GTA ACT TAT ATT CAA GTT CAA GCA CCT ACT GGT ACA TTT
18 His Tyr Ala Pro Val Thr Tyr Ile Gln Val Glu Ala Pro Thr Gly Thr Phe

ATT GCT TCC GGT GTA GTT GTA GGT AAA GAT ACT CTT TTA ACA AAT AAA CAC
35 Ile Ala Ser Gly Val Val Val Gly Lys Asp Thr Leu Leu Thr Asn Lys His

GTC GTA GAT GCT ACG CAC GGT GAT CCT CAT GCT TTA AAA GCA TTC CCT TCT
52 Val Val Asp Ala Thr His Gly Asp Pro His Ala Leu Lys Ala Phe Pro Ser

GCA ATT AAC CAA GAC AAT TAT CCA AAT GGT GGT TTC ACT GCT GAA CAA ATC
69 Ala Ile Asn Gln Asp Asn Tyr Pro Asn Gly Gly Phe Thr Ala Glu Gln Ile

ACT AAA TAT TCA GGC GAA GGT GAT TTA GCA ATA GTT AAA TTC TCC CCT AAT

86 Thr Lys Tyr Ser Gly Glu Gly Asp Leu Ala Ile Val Lys Phe Ser Pro Asn

GAG CAA AAC AAA CAT ATT GGT GAA GTA GTT AAA CCA GCA ACA ATG AGT AAT
103 Glu Gln Asn Lys His Ile Gly Glu Val Val Lys Pro Ala Thr Met Ser Asn

AAT GCT GAA ACA CAA GTT AAC CAA AAT ATT ACT GTA ACA GGA TAT CCT GGT
120 Asn Ala Glu Thr Gln Val Asn Gln Asn Ile Thr Val Thr Gly Tyr Pro Gly

GAT AAA CCT GTA GCA ACA ATG TGG GAA AGT AAA GGA AAA ATC ACT TAC CTC
137 Asp Lys Pro Val Ala Thr Met Trp Glu Ser Lys Gly Lys Ile Thr Tyr Leu

AAA GGC GAA GCT ATG CAA TAT GAT TTA AGT ACA ACT GGT GGT AAT TCA GGT
154 Lys Gly Glu Ala Met Gln Tyr Asp Leu Ser Thr Thr Gly Gly Asn Ser Gly

TCA CCT GTA TTT AAT GAA AAA AAT GAA GTG ATC GGA ATT CAT TGG GGC GGT
171 Ser Pro Val Phe Asn Glu Lys Asn Glu Val Ile Gly Ile His Trp Gly Gly

GTA CCA AAT GAA TTT AAT GGT GCG GTA TTT ATT AAT GAA AAT GTA CGC AAC
188 Val Pro Asn Glu Phe Asn Gly Ala Val Phe Ile Asn Glu Asn Val Arg Asp

TTC TTA AAA CAA AAT ATT GAA GAT ATC CAT TTT GCC AAC GAT GAC CAA CCT
205 Phe Leu Lys Gln Asn Ile Glu Asp Ile His Phe Ala Asn Asp Asp Gln Pro

AAT AAC CCA GAT AAT CCT GAT AAC CCT AAC AAT CCT GAT AAC CCT AAC AAC
222 Asn Asn Pro Asp Asn Pro Asp Asn Pro Asn Asn Pro Asp Asn Pro Asn Asn

CCA GAT GAA CCA AAT AAC CCT GAC AAC CCT AAC AAC CCT GAT AAT CCA GAC
239 Pro Asp Glu Pro Asn Asn Pro Asp Asn Pro Asn Asn Pro Asp Asn Pro Asp

AAT GGC GAT AAC AAT AAT TCA GAC AAT CCA GAT GCA GCT
254 Asn Gly Asp Asn Asn Asn Ser Asp Asn Pro Asp Ala Ala

4. A method according to any one of claims 1, 2 and 3 wherein L-aspartic acid methyl ester is used.

5. A method according to any one of claims 1 to 4 wherein L-phenylalanine methyl ester is used.

6. A method according to any one of the preceding claims wherein the L-aspartic acid alpha ester or alpha amide is also a beta ester or amide, the method further comprising removal of the beta ester or amide to yield L-aspartyl-L-phenylalanine alkyl ester.

7. A method according to any one of the preceding claims wherein the enzyme selected has greater specificity for the L-aspartic acid alpha ester or alpha amide than for the L-phenylalanine alkyl ester.

8. A method according to any one of the preceding claims wherein purified enzyme is used.

9. A method according to any one of the preceding claims wherein the reaction is carried out at about pH 8.

## Patentansprüche

1. Verfahren zur Herstellung von L-Asparaginyl-L-phenylalanin-Alkylestern, umfassend:
a) das Umsetzen von nicht-N-geschütztem L-Asparaginsäure-Alphaester oder L-Asparaginsäure-Alphaamid mit L-Phenylalaninalkylester in einem ein organisches Lösungsmittel enthaltenden Medium in Gegenwart eines Enzyms, eines das Enzym enthaltenden Mikroorganismus, einer enzymhältigen Fraktion eines Mikroorganismus oder eines auf einem festen Träger immobilisierten Enzyms, wobei das genannte Enzym fähig ist, L-Asparaginyl-L-phenylalanin-Alkylester durch Kondensation des nicht-N-geschützten L-Asparaginsäure-Alphaesters oder L-Asparaginsäure-Alphaamids mit dem L-Phenylalaninalkylester zu bilden.

2. Verfahren nach Anspruch 1, worin das ausgewählte Enzym die extrazelluläre Protease von Staphylococcus aureus Stamm V8 oder eine Mutantvariante oder genetisch veränderte Form davon ist, welche dieselbe Funktion beibehält.

3. Verfahren nach Anspruch 1, worin das ausgewählte Enzym einen N-Terminus aufweist, der jenem der folgenden Aminosäuresequenz entspricht:

GTT ATA TTA CCA AAT AAC GAT CGT CAC CAA ATC ACA GAT ACA ACG AAT GGT
1 Val Ile Leu Pro Asn Asn Asp Arg His Gln Ile Thr Asp Thr Thr Asn Gly

CAT TAT GCA CCC GTA ACT TAT ATT CAA GTT CAA GCA CCT ACT GGT ACA TTT
18 His Tyr Ala Pro Val Thr Tyr Ile Gln Val Glu Ala Pro Thr Gly Thr Phe

ATT GCT TCC GGT GTA GTT GTA GGT AAA GAT ACT CTT TTA ACA AAT AAA CAC
35 Ile Ala Ser Gly Val Val Val Gly Lys Asp Thr Leu Leu Thr Asn Lys His

GTC GTA GAT GCT ACG CAC GGT GAT CCT CAT GCT TTA AAA GCA TTC CCT TCT
52 Val Val Asp Ala Thr His Gly Asp Pro His Ala Leu Lys Ala Phe Pro Ser

GCA ATT AAC CAA GAC AAT TAT CCA AAT GGT GGT TTC ACT GCT GAA CAA ATC
69 Ala Ile Asn Gln Asp Asn Tyr Pro Asn Gly Gly Phe Thr Ala Glu Gln Ile

ACT AAA TAT TCA GGC GAA GGT GAT TTA GCA ATA GTT AAA TTC TCC CCT AAT

86 Thr Lys Tyr Ser Gly Glu Gly Asp Leu Ala Ile Val Lys Phe Ser Pro Asn

GAG CAA AAC AAA CAT ATT GGT GAA GTA GTT AAA CCA GCA ACA ATG AGT AAT
103 Glu Gln Asn Lys His Ile Gly Glu Val Val Lys Pro Ala Thr Met Ser Asn

AAT GCT GAA ACA CAA GTT AAC CAA AAT ATT ACT GTA ACA GGA TAT CCT GGT
120 Asn Ala Glu Thr Gln Val Asn Gln Asn Ile Thr Val Thr Gly Tyr Pro Gly

GAT AAA CCT GTA GCA ACA ATG TGG GAA AGT AAA GGA AAA ATC ACT TAC CTC
137 Asp Lys Pro Val Ala Thr Met Trp Glu Ser Lys Gly Lys Ile Thr Tyr Leu

AAA GGC GAA GCT ATG CAA TAT GAT TTA AGT ACA ACT GGT GGT AAT TCA GGT
154 Lys Gly Glu Ala Met Gln Tyr Asp Leu Ser Thr Thr Gly Gly Asn Ser Gly

TCA CCT GTA TTT AAT GAA AAA AAT GAA GTG ATC GGA ATT CAT TGG GGC GGT
171 Ser Pro Val Phe Asn Glu Lys Asn Glu Val Ile Gly Ile His Trp Gly Gly

GTA CCA AAT GAA TTT AAT GGT GCG GTA TTT ATT AAT GAA AAT GTA CGC AAC
188 Val Pro Asn Glu Phe Asn Gly Ala Val Phe Ile Asn Glu Asn Val Arg Asp

TTC TTA AAA CAA AAT ATT GAA GAT ATC CAT TTT GCC AAC GAT GAC CAA CCT
205 Phe Leu Lys Gln Asn Ile Glu Asp Ile His Phe Ala Asn Asp Asp Gln Pro

AAT AAC CCA GAT AAT CCT GAT AAC CCT AAC AAT CCT GAT AAC CCT AAC AAC
222 Asn Asn Pro Asp Asn Pro Asp Asn Pro Asn Asn Pro Asp Asn Pro Asn Asn

CCA GAT GAA CCA AAT AAC CCT GAC AAC CCT AAC AAC CCT GAT AAT CCA GAC
239 Pro Asp Glu Pro Asn Asn Pro Asp Asn Pro Asn Asn Pro Asp Asn Pro Asp

AAT GGC GAT AAC AAT AAT TCA GAC AAT CCA GAT GCA GCT
254 Asn Gly Asp Asn Asn Asn Ser Asp Asn Pro Asp Ala Ala

4. Verfahren nach einem der Ansprüche 1, 2 und 3, worin L-Asparaginsäuremethylester eingesetzt wird.

5. Verfahren nach einem der Ansprüche 1 bis 4, worin L-Phenylalaninmethylester eingesetzt wird.

6. Verfahren nach einem der vorhergehenden Ansprüche, worin der/das L-Asparaginsäure-Alphaester oder -Alphaamid auch ein Betaester oder -amid ist, wobei das Verfahren weiters das Entfernen des Betaesters oder -amids umfaßt, um L-Asparaginyl-L-phenylalaninalkylester zu ergeben.

7. Verfahren nach einem der vorhergehenden Ansprüche, worin das ausgewählte Enzym größere Spezifität für den/das L-Asparaginsäure-Alphaester oder -Alphaamid als für den L-Phenylalaninalkylester aufweist.

8. Verfahren nach einem der vorhergehenden Ansprüche, worin gereinigtes Enzym eingesetzt wird.

9. Verfahren nach einem der vorhergehenden Ansprüche, worin die Umsetzung bei einem pH-Wert von etwa 8 durchgeführt wird.

## Revendications

1. Méthode de production d'esters alkyliques de L-aspartyl-L-phénylalanine comprenant :
a) la réaction,dans un milieu contenant un solvant organique, d'un alpha-ester d'acide L-aspartique non N-protégé ou d'un alpha-amide d'acide L-aspartique avec un ester alkylique de L-phénylalanine, en présence d'une enzyme, d'un microorganisme contenant l'enzyme, d'une enzyme contenant une fraction d'un microorganisme ou d'une enzyme immobilisée sur un support solide, ladite enzyme étant capable de former l'ester alkylique de L-aspartyl-L-phénylalanine par condensation de l'alpha-ester de l'acide L-aspartique non N-protégé ou de l'alpha-amide de l'acide L-aspartique et de l'ester alkylique de L-phénylalanine.

2. Méthode selon la revendication 1, où l'enzyme sélectionnée est la protéase extracellulaire de Staphylococcus aureus souche V8, ou bien a variante mutante ou sa forme génétiquement modifiée qui conserve la même fonction.

3. Méthode selon la revendication 1, où l'enzyme sélectionnée a un N-terminal correspondant à celui de la séquence d'acides aminés :

GTT ATA TTA CCA AAT AAC GAT CGT CAC CAA ATC ACA GAT ACA ACG AAT GGT
1 Val Ile Leu Pro Asn Asn Asp Arg His Gln Ile Thr Asp Thr Thr Asn Gly

CAT TAT GCA CCC GTA ACT TAT ATT CAA GTT CAA GCA CCT ACT GGT ACA TTT
18 His Tyr Ala Pro Val Thr Tyr Ile Gln Val Glu Ala Pro Thr Gly Thr Phe

ATT GCT TCC GGT GTA GTT GTA GGT AAA GAT ACT CTT TTA ACA AAT AAA CAC
35 Ile Ala Ser Gly Val Val Val Gly Lys Asp Thr Leu Leu Thr Asn Lys His

GTC GTA GAT GCT ACG CAC GGT GAT CCT CAT GCT TTA AAA GCA TTC CCT TCT
52 Val Val Asp Ala Thr His Gly Asp Pro His Ala Leu Lys Ala Phe Pro Ser

GCA ATT AAC CAA GAC AAT TAT CCA AAT GGT GGT TTC ACT GCT GAA CAA ATC
69 Ala Ile Asn Gln Asp Asn Tyr Pro Asn Gly Gly Phe Thr Ala Glu Gln Ile

ACT AAA TAT TCA GGC GAA GGT GAT TTA GCA ATA GTT AAA TTC TCC CCT AAT
86 Thr Lys Tyr Ser Gly Glu Gly Asp Leu Ala Ile Val Lys Phe Ser Pro Asn

GAG CAA AAC AAA CAT ATT GGT GAA GTA GTT AAA CCA GCA ACA ATG AGT AAT
103 Glu Gln Asn Lys His Ile Gly Glu Val Val Lys Pro Ala Thr Met Ser Asn

AAT GCT GAA ACA CAA GTT AAC CAA AAT ATT ACT GTA ACA GGA TAT CCT GGT
120 Asn Ala Glu Thr Gln Val Asn Gln Asn Ile Thr Val Thr Gly Tyr Pro Gly

GAT AAA CCT GTA GCA ACA ATG TGG GAA AGT AAA GGA AAA ATC ACT TAC CTC
137 Asp Lys Pro Val Ala Thr Met Trp Glu Ser Lys Gly Lys Ile Thr Tyr Leu

AAA GGC GAA GCT ATG CAA TAT GAT TTA AGT ACA ACT GGT GGT AAT TCA GGT
154 Lys Gly Glu Ala Met Gln Tyr Asp Leu Ser Thr Thr Gly Gly Asn Ser Gly

TCA CCT GTA TTT AAT GAA AAA AAT GAA GTG ATC GGA ATT CAT TGG GGC GGT
171 Ser Pro Val Phe Asn Glu Lys Asn Glu Val Ile Gly Ile His Trp Gly Gly

GTA CCA AAT GAA TTT AAT GGT GCG GTA TTT ATT AAT GAA AAT GTA CGC AAC
188 Val Pro Asn Glu Phe Asn Gly Ala Val Phe Ile Asn Glu Asn Val Arg Asp

TTC TTA AAA CAA AAT ATT GAA GAT ATC CAT TTT GCC AAC GAT GAC CAA CCT
205 Phe Leu Lys Gln Asn Ile Glu Asp Ile His Phe Ala Asn Asp Asp Gln Pro

AAT AAC CCA GAT AAT CCT GAT AAC CCT AAC AAT CCT GAT AAC CCT AAC AAC
222 Asn Asn Pro Asp Asn Pro Asp Asn Pro Asn Asn Pro Asp Asn Pro Asn Asp

CCA GAT GAA CCA AAT AAC CCT GAC AAC CCT AAC AAC CCT GAT AAT CCA GAC
239 Pro Asp Glu Pro Asn Asn Pro Asp Asn Pro Asn Asn Pro Asp Asn Pro Asp

AAT GGC CAT AAC AAT AAT TCA GAC AAT CCA GAT GCA GCT
254 Asn Gly Asp Asn Asn Asn Ser Asp Asn Pro Asp Ala Ala

4. Méthode selon l'une quelconque des revendications 1, 2 et 3, où on utilise l'ester méthylique de l'acide L-aspartique.

5. Méthode selon l'une quelconque des revendications 1 à 4, où on utilise l'ester méthylique de L-phénylalanine.

6. Méthode selon l'une quelconque des revendications précédentes, où l'alpha-ester ou alpha-amide de l'acide L-aspartique est également un bêta-ester ou amide, la méthode comprenant de plus l'élimination du bêta-ester ou amide pour donner l'ester alkylique de L-aspartyl-L-phénylalanine.

7. Méthode selon l'une quelconque des revendications précédentes, où l'enzyme sélectionnée a une plus grande spécificité pour l'alpha-ester de l'acide L-aspartique ou l'alpha-amide que pour l'ester alkylique de L-phénylalanine.

8. Méthode selon l'une quelconque des revendications précédentes, où on utilise une enzyme purifiée.

9. Méthode selon l'une quelconque des revendications précédentes, où la réaction est effectuée à un pH d'environ 8.
